# EUROPEAN PATENT APPLICATION

(11) **EP 4 434 546 A1**
(43) Date of publication of application: **25.09.2024**
(21) Application number: 23758979.1
(22) Date of filing: 03.02.2023
(51) Int. Cl.: A61K 47/54, A61K 31/585, C12N 15/115, A61P 35/00

(54) **NOVEL ACID-SENSITIVE APTAMER TRIPTOLIDE CONJUGATE AND APPLICATION**

(30) Priority: 22.02.2022 CN 202210163765
(71) Applicant: Chengdu University of Traditional Chinese Medicine, Chengdu, Sichuan 610075 (CN)
(72) Inventor: LU, Jun, Chengdu, Sichuan 610075 (CN); DENG, Yun, Chengdu, Sichuan 610075 (CN); CHEN, Yao, Chengdu, Sichuan 610075 (CN); YANG, Jirui, Chengdu, Sichuan 610075 (CN); ZUO, Yi, Chengdu, Sichuan 610075 (CN); LI, Xiao, Chengdu, Sichuan 610075 (CN); REN, Qing, Chengdu, Sichuan 610075 (CN)
(74) Representative: Altmann Stößel Dick Patentanwälte PartG mbB
(86) International application number: PCT/CN2023/074352
(87) International publication number: WO 2023/160354

(57) **Abstract**

The present invention relates to the fields of medicine and chemical industry, and in particular to a conjugate of an aptamer and triptolide (TPL), which is sensitive to weak-acid tumor microenvironment (TME). The conjugate is linked by a linker comprising an acetal ester moiety between 14-OH of triptolide and the aptamer, which is an acid-sensitive moiety with a cleavage condition of (pH = 3.5-6.5) and lower pH sensitivity, thereby making it easier to break in the tumor microenvironment. Based on the characteristic of the aptamer targeting high-expression proteins on tumor cell membranes, this conjugate delivers triptolide to tumor cells, which enters lysosomes by mediating endocytosis; based on the characteristics of lysosomal acidic environment, intact triptolide is released in lysosomal acidic environment due to the rupture of the acetal ester moieties, thereby producing target killing effects on tumor cells. This overcomes the following shortcomings, that is, the acid sensitivity of the linker for the aptamer-triptolide conjugate in the prior art is low, which is not easily broken in the *in vivo* environment of cancer cells, resulting in insufficient targeted release of triptolide, and thus the inhibitory effects on cancer cells are not significant.

## Description

### Technical field

The present invention relates to the fields of medicine and chemical industry, and mainly relates to a conjugate of an aptamer and triptolide (TPL), which is sensitive to weak-acid tumor microenvironment (TME); specifically, relates to a novel acid-sensitive conjugate of an aptamer and triptolide, as well as the uses thereof.

### Background technology

Triptolide is an epoxy diterpene lactone extracted from the roots, leaves, flowers, and fruits of *Tripterygium wilfordii,* a plant in the family Celastraceae. Triptolide, together with triptoline, wilforine, wilfogine, wilfotrine, wilfozine, wilfordsine and other alkaloids, consists of the main active ingredients of *T. wilfordii* extracts, which is insoluble in water, but soluble in methanol, dimethylsulfoxide, anhydrous ethanol, ethyl acetate, chloroform, etc. Triptolide has broad-spectrum anti-tumor activity, can inhibit the proliferation of various tumor cells, and induce cell apoptosis and autophagy. The anti-tumor activity of triptolide is superior to traditional anticancer drugs such as doxorubicin and paclitaxel, which can effectively inhibit the proliferation of tumor cells at extremely low concentrations (2-10 ng/mL). In addition, triptolide can also be effective against drug-resistant tumors, and increase the sensitivity of tumor cells to other anti-tumor medicaments, and play synergistic effects when combined with chemotherapeutic medicaments and ionizing radiation.

Cancer has become one of the main threats to people's life safety. One of the differences between tumor cells and normal cells is the abnormal expression of certain proteins in tumor cells. For example, mucin 1 (MUC1) is abnormally expressed on the surface of breast cancer cells (MCF-7) and their drug-resistant cell lines; nucleolin and epidermal growth factor receptor (EGFR) are also abnormally expressed in tumor cells, the former is abnormally expressed in triple negative breast cancer MDA-MB-231 cell lines, lung cancer A549 cell lines, and colon cancer HCT116 cell lines, and the latter is usually used as a target for treatment of lung cancer.

Aptamer is a single-stranded oligonucleotide which binds to a specific target using its advanced structure due to self-folding, and mainly refers to a fragment of DNA (deoxyribonucleic acid), RNA (ribonucleic acid), or modified DNA and RNA. Aptamers can bind to target molecules by 3D complementary conformation, with similar affinity and specificity to antibodies. They also have advantages such as good water solubility, low immunogenicity, easy production, low cost, and good stability, and have been clinically proven to be safe. Due to the ability to achieve specific delivery at the cellular level, aptamers have been widely used as targeting molecules in drug delivery research. For example, mucin 1 (MUC1) aptamer (Apt) can specifically bind to MUC1 with high affinity, and its sequence is short, making it easy to synthesize and purify; aptamer AS 1411 can specifically bind to the highly expressed nucleolin on the surface of tumor cells, and the nucleolin on the membrane can promote the macropinocytosis of AS1411 by tumor cells, increasing uptake, while AS 1411 can form a G-quadruplex structure to enhance its stability; aptamer E07 can specifically bind to the epidermal growth factor receptor (EGFR).

Aptamers Apt, AS1411, and E07 all have the characteristics of good water solubility, strong stability, and targeting high-expression proteins in tumor cells, thereby making them become carriers suitable for drug transport. As a broad-spectrum anti-cancer drug, triptolide has a strong anti-tumor activity, but its toxic side actions are also significant, and its water solubility is poor. Therefore, the modified triptolide is allowed to conjugate with an aptamer, so as to transport triptolide to a specific tumor site with the targeting effects of an aptamer, bind with highly expressed receptors on the cell membrane, and thus triptolide is able to have target killing effects on tumors while avoid its killing on normal cells.

In addition to certain proteins specifically high expressed in tumor cells, due to the fact that tumor cells need to infiltrate surrounding tissues to achieve proliferation and metastasis, the content of lysosomes in tumor tissues is much higher than that in normal tissues. Lysosomes are important degraders in eukaryotic cells, and maintain the metabolic stability, of which one feature is the acidic environment (pH = 3.5-5.5) within the organelles.

In the prior art, the vinyl ether moiety is used as an acidic linker to connect triptolide and an aptamer. During the research process, it was found that the vinyl ether moiety can only be cleaved at a pH of <4. This results in a lower releasing rate of the aptamer-triptolide conjugate formed with this linker in tumor cells, and a worse inhibitory effect on cancer cells.

### Summary of the invention

The present invention is intended to overcome the shortcomings of the aptamer-triptolide conjugate in the prior art, that is, the acid sensitivity of the linker is low and not easily ruptured in the *in vivo* cancer cell environment, and thus the targeted release of triptolide is insufficient, which further cause the inhibitory effect on cancer cells is not significant. Thereby, the present invention provides a new moiety as the linker between triptolide and an aptamer, and specifically, provides a novel acid-sensitive conjugate of an aptamer and triptolide, and uses thereof.

In order to achieve the above-mentioned invention objectives, the present invention provides the following technical solutions:

An acid-sensitive conjugate of an aptamer and triptolide, and the general structural formula of the conjugate is as follows: 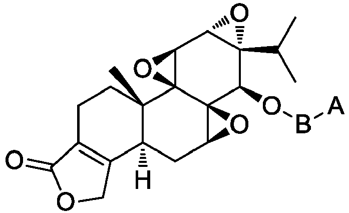 wherein, A is an aptamer; B is a linker between triptolide and an aptamer, and the linker comprises an acetal ester moiety formed at the 14-OH position.

The technical solution of the present invention discloses an aptamer-triptolide conjugate, in which 14-OH of triptolide is linked to the aptamer via a linker (an acetal ester moiety) formed by acetal and ester groups. The linker is acid sensitive, and its cleavage condition is (pH = 3.5-6.5). Compared with the linker (breaking at a pH of < 3.5) in the prior art, its pH sensitivity is lower, and it is easier to break in the tumor microenvironment. Based on the characteristics that aptamers target the high expression proteins on the membrane of tumor cells, the conjugate can specifically deliver triptolide to tumor cells, and then triptolide is internalized into lysosomes; based on the characteristics of lysosomal acidic environment, the acetal moiety is cleaved in lysosomal acidic environment, so as to release triptolide, which targets and kills tumor cells.

As the preferred technical solution of the present invention, the general structural formula of the linker B is as follows:

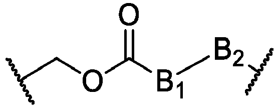

Research on the structure-activity relationship of triptolide has revealed that its 14-OH is both an active site and a modification site. Linker B is linked to 14-OH of triptolide and forms an acetal moiety, which can block the toxic effects of triptolide on normal tissues. At the same time, triptolide is released in an acidic lysosomal environment, thereby achieving precise cancer treatment.

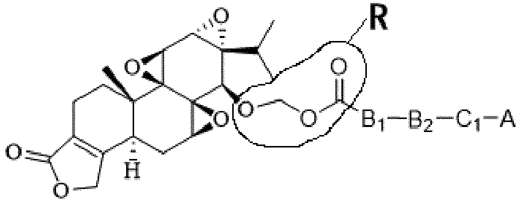

As shown in the above general structural formula, the circled position is a new linker formed between linker B and 14-OH of triptolide, which is defined as an acetal ester moiety.

The following scheme shows the reaction for the acid-sensitive conjugate of an aptamer and triptolide.

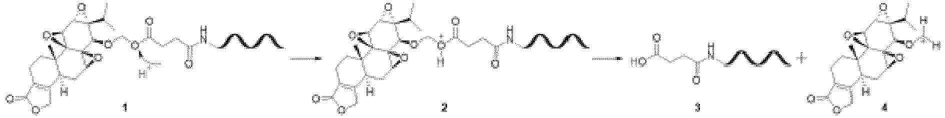

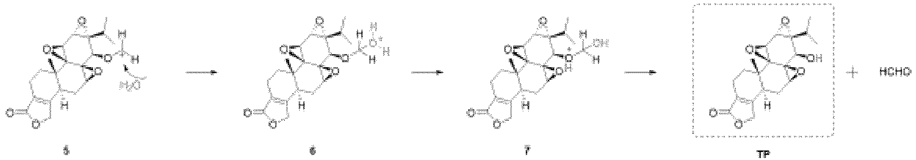

Cleavage mechanism: in an acidic environment, H⁺ attacks the oxygen at the ester end of the acetal ester moiety in the aptamer-triptolide conjugate (1), and forms an unstable oxonium salt intermediate (2), leading to further cleavage and the formation of a moiety containing the aptamer and the linker (3), as well as an extremely unstable triptolide intermediate containing carbocation (4). Then, a molecule of water combines with the carbocation of the triptolide intermediate (5), and the carbocation proton is transferred to the oxygen in the water (6). Then, the proton is further transferred to the oxygen of triptolide, forming an unstable triptolide hemiacetal structure (7). The C-O bond in the hemiacetal structure breaks to produce triptolide and formaldehyde.

Further preferably, the general structural formula of B1 is as follows: 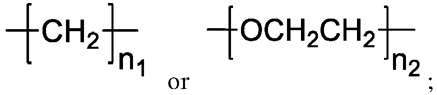
wherein, 1≤n₁≤100; preferably, 1≤n₁≤50; more preferably, 1≤n₁≤20;
wherein, 1≤n₂≤100; preferably, 1≤n₂≤50; more preferably, 1≤n₂≤20.

In the present embodiment, for B₁, wherein 1 ≤ n₁(n₂) ≤ 100, the regulatory design of alkyl or alkoxy chains can interfere with the binding of triptolide-aptamer conjugate to the target protein, and appropriately increasing the contact area between the linker and the tumor microenvironment improves the release rate of triptolide. However, the synthesis cost of longer alkane chains is high, and the yield is low. Therefore, the preferred is 1 ≤ n₁ (n₂) ≤ 50, and the more preferred is 1 ≤ n₁ (n₂) ≤ 20.

As the preferred technical solution of the present invention,

The general structural formula of B₂ is as follows:

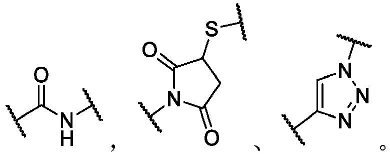

As the preferred technical solution of the present invention, the aptamer includes an aptamer modifier, and the general structural formula of the aptamer modifier is as follows: A₁-C₁-; the structure of C₁ includes: 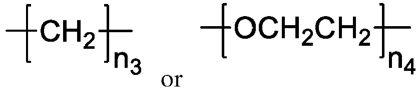 or a combination thereof;
wherein, 1≤n₃≤100; preferably, 1≤n₃≤50; more preferably, 1≤n₃≤20;
wherein, 1≤n₄≤100; preferably, 1≤n₄≤50; more preferably, 1≤n₄≤20.

In the present embodiment, for C₁, wherein 1 ≤ n₃ ≤ 100, the design of alkyl or alkoxy chains can interfere with the binding of triptolide-aptamer conjugate to the target protein, and meanwhile, the contact area between the linker and the tumor microenvironment can be increased, so as to improve the release rate of triptolide. However, the synthesis cost of longer alkane chains is high, and the yield is low. Therefore, the preferred is 1 ≤ n₃ (n₄) ≤ 50, and the more preferred is 1 ≤ n₃ (n₄) ≤ 20.

As a preferred technical solution of the present invention, the conjugate is obtained by reacting a derivative of triptolide with a aptamer modifier. Specifically, the derivative of triptolide is synthesized by reacting 14-OH of triptolide with the linker B in an organic solvent, which is a triptolide derivative containing an acetal ester moiety formed with 14-OH of triptolide.

As a preferred technical solution of the present invention, the general structural formula of the conjugate is as represented by the following structure:

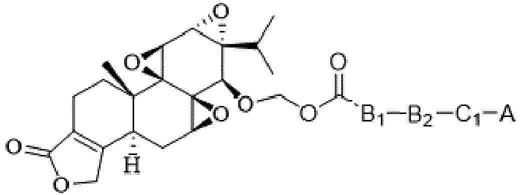

The conjugate is obtained by any of the substitution, cyclization, or addition reactions of the B2 functional group in the triptolide analogues, specifically the substitution reaction between amino and carboxyl groups; the addition reaction between thiol and maleimide, or the cyclization reaction between azide and alkynyl groups.

The aptamer is selected from the group consisting of AS1411, Pegaptanib, Sgc8c, A10, DNAaptamer, RNAaptamer, CL4, Apt, and E07.

The sequence of aptamer is as follows:
AS 1411: GGTGGTGGTGGTTGTGGTGGTGGTGG;
Pegaptanib: GCGAACCGAUGGAAUUUUUGGACGCUCGC;
Sgc8c: ATCTAACTGCTGCGCCGCCGGGAAAATACTGTACGGTTAGA;
A10: GGGAGGACGAUGCGGAUCAGCCAUGUUUACGUCACUCCUUGU CAAUCCUCAUCGGCAGACGACUCGCCCGA;
DNA aptamer: GGGAGACAAGAATAAACGCTCAA-(25N)- TTCGACAGGA GGCTCACAACAGGC;
RNA aptamer: GGGGGCAUACUUGUGAGACUUUUAUGUCACCCCC;
CL4: GCCUUAGUAACGUGCUUUGAUGUCGAUUCGACAGGAGGC;
Apt: GCAGTTGATCCTTTGGATACCCTGG;
E07: GGACGGAUUUAAUCGCCGUAGAAAAGCAUGUCAAAGCCGGAA CCGUCC.

As a preferred technical solution of the present invention, provided is a method for preparing the acid-sensitive conjugate of an aptamer and triptolide, comprising the following steps:
Step 1: A triptolide analogue is prepared, wherein the 14-OH position of triptolide is linked to an acetal ester moiety;
Step 2: The aptamer is modified to obtain the aptamer modifiers A₁-C₁;
Step 3: The conjugate is synthesized by linking a triptolide analogue and an aptamer modifier obtained in step 2 with the linker B₂.

Preferably, the acid-sensitive triptolide-aptamer conjugate can be prepared by those skilled in the art in combination with the content of the present invention and attempts made in the art;

14-OH of triptolide is blocked by forming an acetal ester functional group with a linker, to obtain triptolide derivatives; at the same time, the aptamer is first modified with alkane or alkoxy chains; then, based on the type of terminal functional groups of the triptolide derivatives, the end of the aptamer connecting the alkane or alkoxy chain is modified with a functional group; finally, the derivative of triptolide is reacted with the modified aptamer in the presence of catalytic reagents, using the amino or thiol or azide groups in the aptamer as well as the carboxyl or maleimide or azide groups in triptolide analogues, to obtain a conjugate. After the two are linked, they form the linker B2 mentioned above.

The acid-sensitive triptolide-aptamer conjugate mentioned above is used in the manufacturer of anti-tumor medicaments.

### Compared with the prior art, the beneficial effects of the present invention are as follows:

1. In the aptamer-triptolide conjugate provided in the present invention, the linker between triptolide and the aptamer contains an acetal ester moiety, that is, the linker between 14-OH of triptolide and the aptamer is that having an acetal ester moiety. This linker is an acid-sensitive type, with a cleavage condition of (pH = 3.5-6.5), lower pH sensitivity, good acid responsiveness, which is easier to break in the tumor microenvironment.
2. For the aptamer-triptolide conjugate provided in the present invention, the *in vitro* activity study of AS 1411-TP shows that it has anti-tumor effects comparable to triptolide on various tumor cells, and the linkage of an aptamer does not affect the anti-tumor effect of triptolide. At the same time, it is demonstrated that the acid-sensitive linker responsive to the tumor microenvironment can break in tumor cells, releasing triptolide.
3. For the aptamer-triptolide conjugate provided in the present invention, the active group of triptolide is blocked by an aptamer in a normal cellular environment, so that triptolide cannot be released in intact forms. Thus, compared with pure triptolide, the cytotoxicity of the conjugate is very low, indicating that the aptamer will not damage normal cells after entering the human body. The toxicity to the human body is relatively low.
4. The aptamer-triptolide conjugate provided in the present invention has good tumor-targeting properties, and can avoid other tissues, with the tumor-targeting effect; the intravenous administration experiments have shown that AS 1411-TP has good anti-tumor effects *in vivo,* and the linker used to connect triptolide and an aptamer has played a significant role *in vivo,* which ensures that the conjugate does not break in normal tissues and has low toxicity; while, when the conjugate cleaves in tumor tissue, it releases triptolide, which plays a therapeutic role.

### Description of Figures

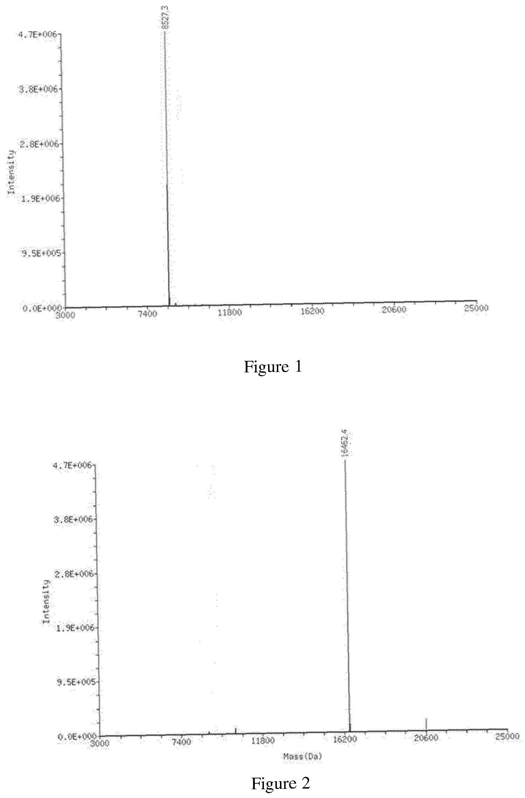

Figure 1. The MS of compound **5;**
Figure 2. The MS of compound **7;**
Figure 3. The MS of compound **9;**
Figure 4. HPLC analysis of the stability for the conjugate (AS1411-TP) in serum at different time points;
Figure 5. HPLC analysis of the stability for the conjugate (E07-TP) in serum at different time points;
Figure 6. HPLC analysis of the release of triptolide from the conjugate (AS1411-TP) at different pH values;
Figure 7. HPLC analysis of the release of triptolide from the conjugate (E07-TP) at different pH values;
Figure 8. HPLC analysis on the release of triptolide from the conjugate (conjugate AS1411-TP linked with a vinyl ether linker) at different pH values;
Figure 9. The chemical formula of AS 1411-TP-1;
Figure 10. The inhibitory curves of AS 1411-TP, TP, and AS 1411 on colon cancer HCT116 cell lines;
Figure 11. The inhibitory curves of AS 1411-TP, TP, and AS 1411 on lung adenocarcinoma A549 cell lines;
Figure 12. The inhibitory curves of AS 1411-TP, TP, and AS 1411 on breast cancer MDA-MB-231 cell lines;
Figure 13. The inhibitory curves of AS1411-TP, TP, and AS1411 on pancreatic cancer PANC-1 cell lines;
Figure 14. The inhibitory curves of AS 1411-TP, TP, and AS 1411 on liver cancer HepG2 cell lines;
Figure 15. Toxic effect curve of AS 1411-TP, TP, and AS 1411 on human embryonic kidney cell HEK293;
Figure 16. Toxic effect curve of AS 1411-TP, TP, and AS 1411 on human normal liver cell LO2;
Figure 17. The distribution of AS 1411-TP, TP, and AS 1411 in different tissues of colon cancer models in tumor-bearing mice;
Figure 18. The distribution of AS 1411-TP, TP, and AS 1411 in different tissues of breast cancer models in tumor-bearing mice;
Figure 19. The distribution of AS 1411-TP, TP, and AS1411 in different tissues of lung cancer models in tumor-bearing mice;
Figure 20. The image of AS 1411-TP, TP, and AS 1411 against colon cancer xenograft tumors in mice;
Figure 21. The image of AS 1411-TP, TP, and AS1411 against breast cancer xenograft tumors in mice;
Figure 22. The image of AS 1411-TP, TP, and AS 1411 against lung cancer xenograft tumors in mice;
Figure 23. The chart for data analysis of AS 1411-TP, TP, and AS 1411 *in vivo* stability test.

### Examples

By following description of Experimental Example and specific Examples, the present invention is further illustrated. However, it should not be construed that the scope of the above subject matter of the present invention is limited to the following examples. The techniques realized based on the above content are all within the scope of the present invention.

### Example 1

This example provided an acid-sensitive aptamer-triptolide conjugate, abbreviated as Apt-TP. The structural formula and reaction route of the conjugate are as follows:
Aptamer 1 is an amino-modified Apt synthesized by the nucleic acid synthesizer AKTAOligopilot100 system; specifically, aptamer 1: NH₂-(CH2)₆-5'GCAGTTGATCCTTTGGATACCCTGG3'MS:7843;

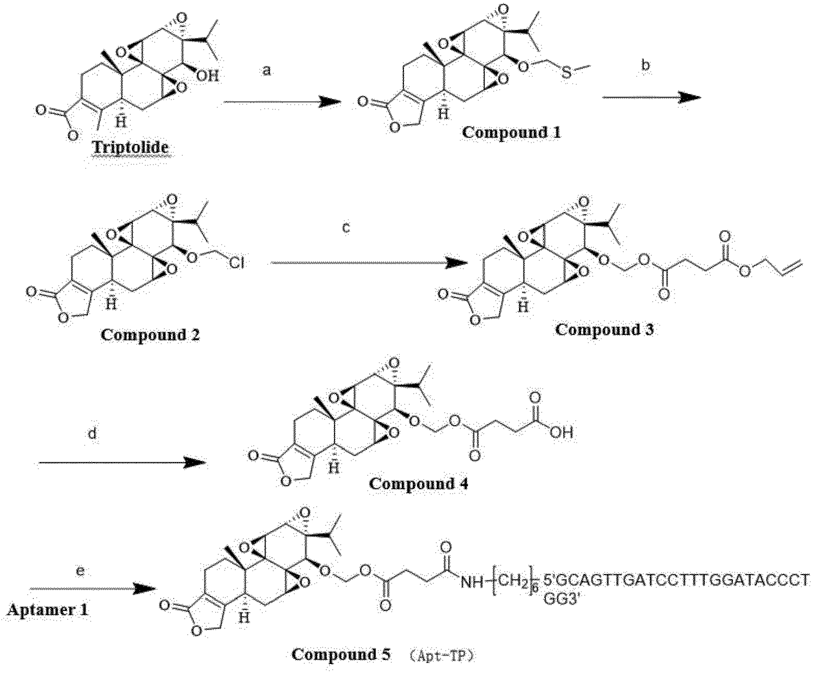

### Specifically, provided is the preparation procedures of compound 5 provided in Example 1 above:

### Compound 1:

1 eq of triptolide and 8 eq of dimethyl sulfide were dissolved in acetonitrile in an ice bath, to which was added 4 eq of benzoyl peroxide in four portions over 40 minutes. Under nitrogen protection, the mixture was stirred in an ice bath for 1 hour, and then stirred at room temperature for 2 hours. The sample was collected and subjected to TLC and LC-MS detection, that indicated the reaction was no longer in progress. The mixture was diluted with ethyl acetate and washed with 10*%* Na₂CO₃ and brine. The organic phase was dried over anhydrous Na₂SO₄, filtered, and concentrated to obtain the crude product, which was purified by silica gel column to obtain pure compound **1** (MS: 420.2) as a white powder.

### Compound 2:

1 eq of compound **1** was dissolved in DCM, to which was added 1.5 eq of SO₂Cl₂, and then the mixture was stirred at room temperature for 2 hours. The solvent was directly rotatory evaporated to dry, to obtain crude compound **2** (MS: 408.3), which was directly used in the next step.

### Compound 3:

1.0 eq of compound **2** was dissolved in 20 ml of DCM, to which was added 0.7 eq of 18-crown-6 ether and 1.7 eq of 4-(allyloxy)-4-oxobutyric acid sodium salt, and then the mixture was stirred at room temperature for 2 hours under nitrogen protection. The sample was collected and subjected to TLC and LC-MS detection, which indicated the reaction was completed. Then, the reaction solution was diluted with DCM, and saturated NaHCO₃ solution was added. The organic phase was collected, and washed twice with saturated brine. The organic phase was dried over anhydrous Na₂SO₄, filtered, and concentrated to obtain the crude product, which was purified by silica gel column to obtain pure compound **3** (MS: 530.3).

### Compound 4:

1.0 eq of compound **3** and 2.5 eq of 1,3-dimethylbarbituric acid were dissolved in a mixed solvent of dichloromethane and ethyl acetate (1:1, v/v), to which was added 0.1 eq of tetrakis(triphenylphosphine)palladium. After the entire reaction system was allowed to react at room temperature for 2 hours, the organic solvent was removed under reduced pressure at 40 °C. The crude product obtained was purified by column chromatography, to provide compound **4** (MS: 490.4).

### Compound 5:

1 eq of synthesized amino-modified Apt (aptamer 1) was dissolved in deionized water, to which was added 100 eq of NaHCO₃ buffer at pH 9, followed by addition of DMT-MM aqueous solution (100 eq), and finally the solution of compound **4** in DMSO was added at 70 eq. After the reaction was shaken at 37 °C on a shaker for 24 hours, the reaction was detected with HPLC, indicating completion of the reaction. After that, 2.5 volumes of anhydrous ethanol were added. The resultant solution was stirred well, placed on dry ice for 1 hour, and then removed to centrifuge at high-speed and low temperature. The precipitate was collected and dissolved in dd-H₂O to form a solution. Finally, the collected product was purified using RP-HPLC and freeze-dried using a freeze-dryer, to obtain pure compound **5** (MS: 8527.3) as white powder.

### Example 2

This example provides an acid-sensitive aptamer-triptolide conjugate, designated as E07-TP, and the structural formula and reaction route of the conjugate were as follows:
Aptamer 2 is E07 produced via nucleic acid synthesizer AKTAOligopilot100system, in which the thiol is modified with PEG;
Specifically, aptamer 2: SH-CH₂CH₂-(OCH₂CH₂)₁₀-O-5'GGACGGAUUUAAUCGCCGUAGAAAAGCAUGUCAAAGCC GGAACCGUCC3' MS:15990.5; 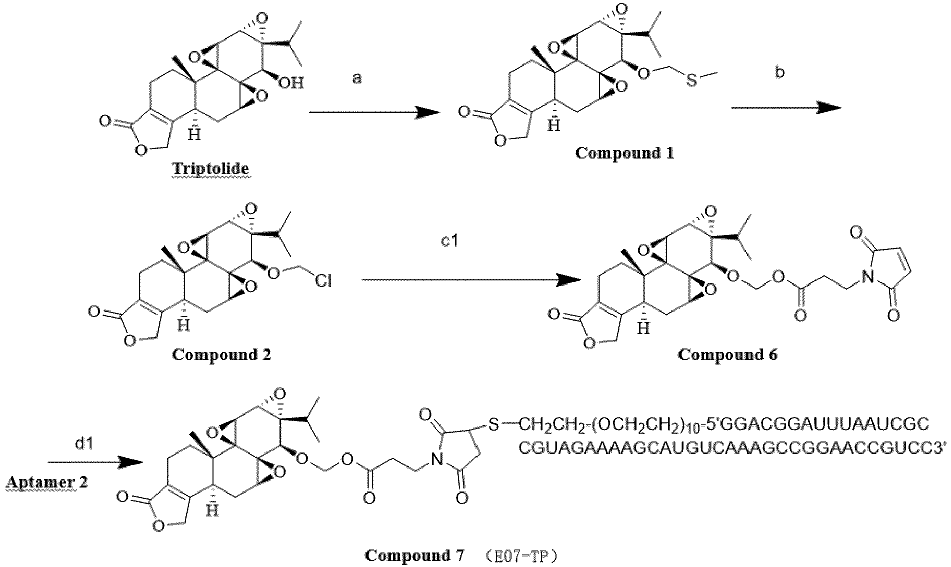 wherein, the synthetic procedure of conjugate 7 included the following steps:
   Compounds **1** and **2** were prepared using the same method as in Example 1, and would not be further elaborated here;

### Compound 6:

1.0 eq of compound **2** was dissolved in DCM, to which was added 0.7 eq of 18-crown-6 ether and sodium 3-maleimidopropionate, and then the mixture was stirred at room temperature for 2 hours under nitrogen protection. The sample was collected and subjected to TLC and LC-MS detection, which indicated the reaction was completed. Then, the reaction solution was diluted with DCM, and then saturated NaHCO₃ solution was added. The organic phase was collected, and washed twice with saturated brine. The organic phase was dried over anhydrous Na₂SO₄, filtered, and concentrated to obtain the crude product, which was purified by silica gel column to obtain pure compound **6** (MS: 541.2).

### Compound 7:

1 eq of sulfhydryl-modified E07 (aptamer 2) was dissolved in a Na₂CO₃/NaHCO₃ buffer at pH = 7.4, to which was added 50 eq of TECP for activation, and then the solution was shaken for 2 hours at 4 °C. The reaction solution was added to the solution of compound **6** (100 eq) in DMF, and then the reaction system was allowed to react at room temperature for 24 hours. After the reaction was completed, the product was precipitated by adding ethanol (1:2.5, v/v), which was purified with RP-HPLC, to obtain the target product compound **7** (MS: 16462.4).

### Example 3

This example provides an acid-sensitive aptamer-triptolide conjugate, designated as AS 1411-TP, and the structural formula and reaction route of the conjugate were as follows:
Aptamer 3 is AS1411 produced via nucleic acid synthesizer AKTAOligopilot100system, in which the azido is modified with PEG;
Aptamer 3 is: N₃-CH₂CH₂-(OCH₂CH₂)₃-O-5GGTGGTGGTGGTTGTGGTGGTGGTGG3' MS:8553.4 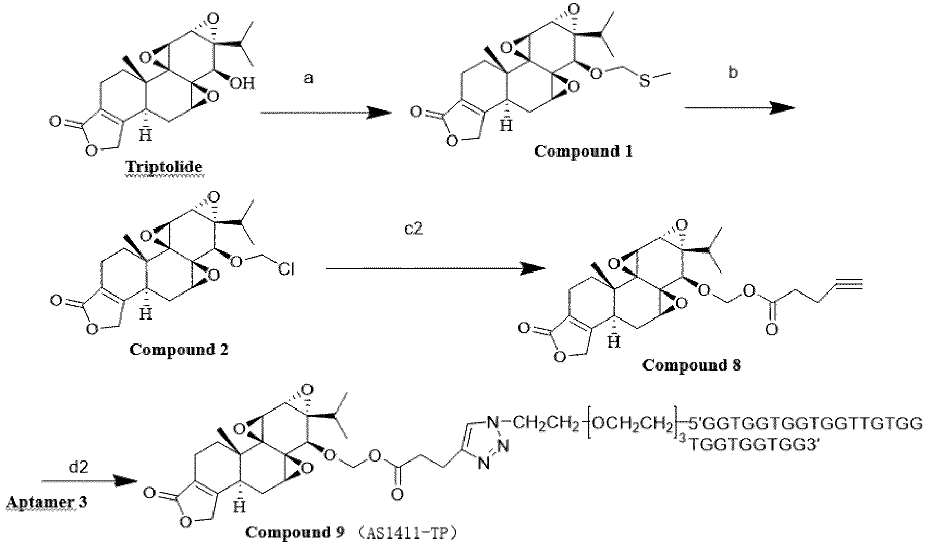 wherein, the synthetic procedure of conjugate 7 included the following steps:
   Compounds **1** and **2** were prepared using the same method as in Example 1, and would not be further elaborated here;

### Compound 8:

1.0 eq of compound **2** was dissolved in DCM, to which was added 0.7 eq of 18-crown-6 ether and 3 eq of sodium 4-pentynoate, and then the mixture was stirred at room temperature for 2 hours under nitrogen protection. The sample was collected and subjected to TLC and LC-MS detection, which indicated the reaction was completed. Then, the reaction solution was diluted with DCM, and then saturated NaHCO₃ solution was added. The organic phase was collected, and washed twice with saturated brine. The organic phase was dried over anhydrous Na₂SO₄, filtered, and concentrated to obtain the crude product, which was purified by silica gel column to obtain pure compound **8** (MS: 470.4).

### Compound 9:

1 eq of aptamer 3, in which the azido was modified with PEG, was dissolved in a Na₂CO₃/NaHCO₃ buffer at pH = 7.4, to which were added 3 eq of trimethylamine and the same volume of tert-butanol, and then the reaction solution was added to the solution of compound **8** (100 eq) in DMF, followed by addition of CuSO₄.5H₂O (50 eq) and sodium ascorbate (50 eq). The reaction system was allowed to react at room temperature for 24 hours under nitrogen protection. After completion of the reaction, the product was precipitated by adding ethanol (1:2.5, v/v), which was purified with RP-HPLC, to obtain the target product compound **9** (MS: 9023.4).

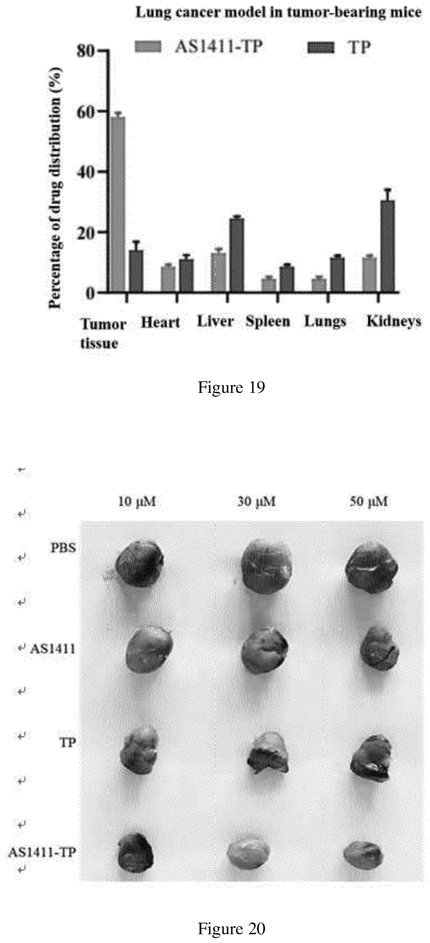

The MS of the conjugate prepared in Examples 1-3 are shown in Figures 19-21.

### I. Study on the serum stability and the sensitivity to the weak-acid microenvironment of conjugates

In order to verify the serum stability and the sensitivity to the weak-acid microenvironment of the conjugate according to the present invention, HPLC detection was performed separately. The detailed procedures were as follows:
Materials: AS1411-TP conjugate (with a linker containing an acetal ester moiety), E07-TP conjugate (with a linker containing an acetal ester moiety), AS1411-TP-1 conjugate (with a linker containing a vinyl ether moiety), mouse serum collected from BALB/c mice, if-, 7-8 weeks old (SiPeiFu (Beijing) Biotechnology Co., Ltd).

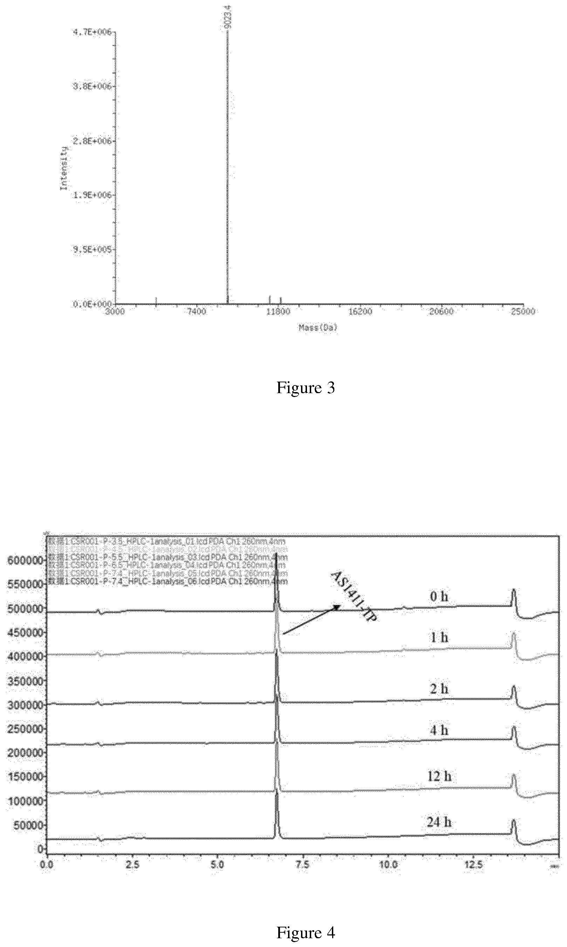

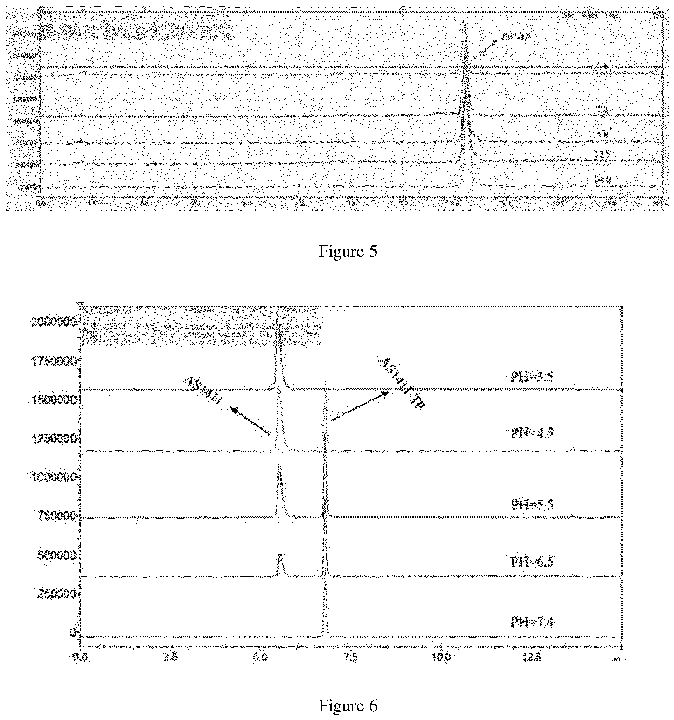

Experimental methods: AS1411-TP conjugate (with a linker containing an acetal ester moiety, 2 mg) and E07-TP conjugate (with a linker containing an acetal ester moiety, 2 mg) were dissolved in 100 µL of mouse serum, to simulate the stability of conjugates in the blood circulation system, and six parallel groups were set up. The drug and serum were incubated in a 37 °C incubator, and then the drug was filtered at 0 h, 1 h, 2 h, 4 h, 12 h, and 24 h, and the content of the conjugate was determined using HPLC, after dissolution for different times. The detection results are shown in Figures 4 and 5; as shown in Figures 4 and 5, within 24 hours, only the conjugate was detected in HPLC, and there was no cleavage of the conjugate. This indicated that the aptamer-triptolide conjugate of the present invention (with a linker containing an acetal moiety) can remain stable in serum for a long time.

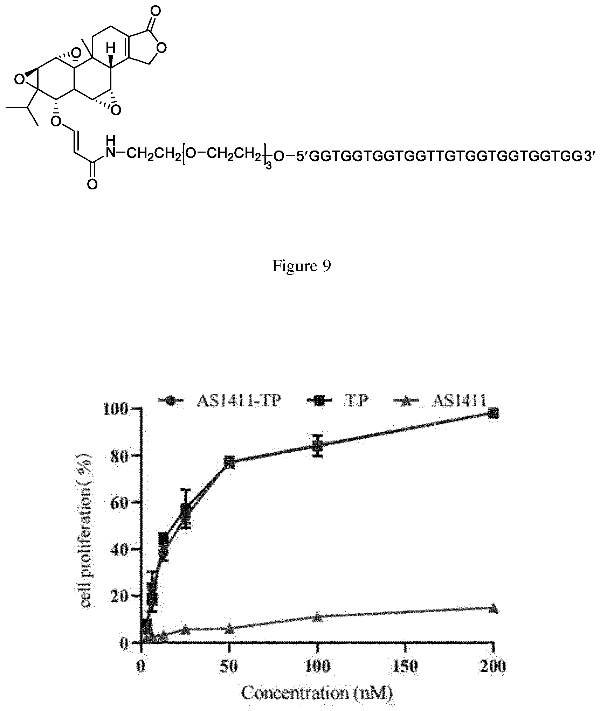

The tumor microenvironment is a weak-acid environment, and the sensitivity of conjugates to the weak-acid microenvironment determined the therapeutic effect of drugs. AS1411-TP-1 conjugate (with a linker containing an acetal ester moiety, 2 mg), AS1411-TP conjugate (with a linker containing an acetal ester moiety, 2 mg), and E07-TP conjugate (with a linker containing an acetal ester moiety, 2 mg) were dissolved in 100 µL of PBS buffer at different pH values, to simulate the tumor microenvironment. After 2 hours, HPLC was used to determine the changes in the content of conjugates under different pH conditions. Among them, AS1411-TP conjugate (with a linker containing an acetal ester moiety) was used as the control group and named AS1411-TP-1. The chemical formula of AS 1411-TP-1 is shown in Figure 9.

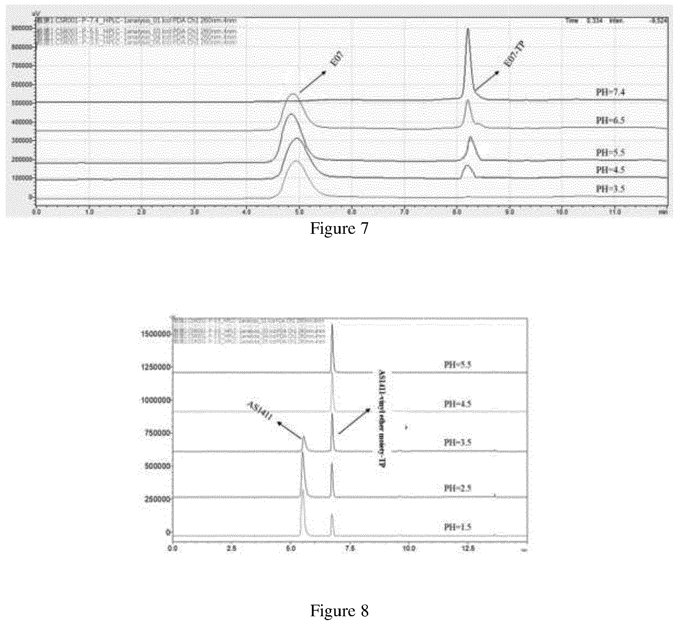

The detection results are shown in Figures 6, 7, and 8. Figure 6 shows the HPLC of AS1411-TP conjugate, Figure 7 shows the HPLC of E07-TP conjugate, and Figure 8 shows the HPLC of AS1411-TP-1. From the above three figures, the conjugate with a linker containing an acetal ester moiety began to break at pH 6.5, and the conjugate completely cleaved at pH 3.5, while the conjugate with a linker containing an ether bond only started to degrade at pH 3.5. This indicated that compared to the vinyl ether moiety, the acetal ester moiety is more sensitive to pH values and more prone to cleavage in the tumor microenvironment to release triptolide, suggesting that the conjugate linked by an acetal ester moiety has better responsiveness to weak-acid microenvironment in tumors.

The above experiments on the serum stability and the sensitivity to weak-acid microenvironment fully demonstrated that the acetal ester moiety in the conjugate of the present invention not only had serum stability, but also had low requirements for cleavage of linkers in acidic environments. When the pH value is around 6, the cleavage of the linker can be achieved, releasing triptolide and playing pharmacological effects. The release rate of triptolide was greatly increased, thereby allowing for more efficient utilization of triptolide.

The nucleolin aptamer AS 1411 was a highly water-soluble single-stranded oligonucleotide, which could specifically bind to nucleolin proteins that were highly expressed on the tumor cell membranes. Therefore, taking the nucleolin aptamer-triptolide conjugate in Example 3 as an example, the *in vitro* activity, the *in vitro* toxicity, the *in vivo* distribution, the *in vivo* activity, the *in vivo* stability and the same were studied to fully characterize and verify the performance of the linker in the conjugate of the present invention as well as the feasibility of inhibiting cancer cells *in vivo.*

### II. In vitro activity study of AS1411-TP

Material: Aptamer-triptolide conjugate (AS1411-TP). Triptolide (TP) is provided by Shanghai Yaji Biotechnology Co., Ltd; the aptamer (AS1411) was obtained from HitGen Inc., Chengdu; trypsin, thiazole blue (MTT) (Biofloxx, Germany); dimethyl sulfoxide (DMSO) (MPBiomedicals, France); fetal bovine serum (FBS) (Newzerum, New Zealand); DMEM medium, RPMI1640 medium, culture dish, 96 well plate (Corning, USA); colon cancer HCT116 cells, breast cancer MDA-MB-231 cells, non-small cell lung cancer A549 cells, pancreatic cancer PANC-1 cells, and liver cancer HepG2 cells were purchased from the cell bank of the Chinese Academy of Sciences (Shanghai) and stored in our laboratory.

### Experimental procedures:

### 2.1 Cell recovery

Tumor cell lines HCT116, MDA-MB-231, A549, PANC-1, and HepG2 were taken out from a liquid nitrogen tank, and placed in a 37 °C water bath. After the frozen cells melted into cell suspension, the suspension was centrifuged at a speed of 1000 r/min for 3 min, and then the supernatant was discarded, followed by adding 1 ml of fresh complete culture medium containing 10% fetal bovine serum and 1% double antibody. The cells were mixed well, and then 5 ml of medium was added. The cells were gently and repeatedly blown with a pipette to mix well. Finally, the cell suspension was transferred to a 10 mm culture dish, and after labelling the cell name, cell passages, recovery date, and operator's name, the cells were placed in an incubator for cultivation (HCT116, PANC-1, and HepG2 cell lines were cultured in DMEM medium, while MDA-MB-231 and A549 cell lines were cultured in RPMI1640 medium).

### 2.2 Cell passage

HCT116, MDA-MB-231, A549, PANC-1, and HepG2 cells in good growth condition were selected, and then the medium was removed. The cells were washed with 1 ml of sterile PBS, to which was added 1.5 ml of trypsin to digest the cells, and after about 2 minutes, the cells become round and detached. 1.5 ml of medium was added to terminate digestion, and then the cells were transferred to a centrifuge tube, and centrifuged at 1000 r/min for 3 min. The supernatant was discarded, 1 ml of fresh medium was added for resuspension, and then additional fresh medium was added in a ratio of 1:3 (v/v), followed by dividing into three culture dishes. The passage date and number were marked, and then the cells were placed in an incubator for cultivation.

### 2.3 Determination of AS1411-TP activity by MTT experiment

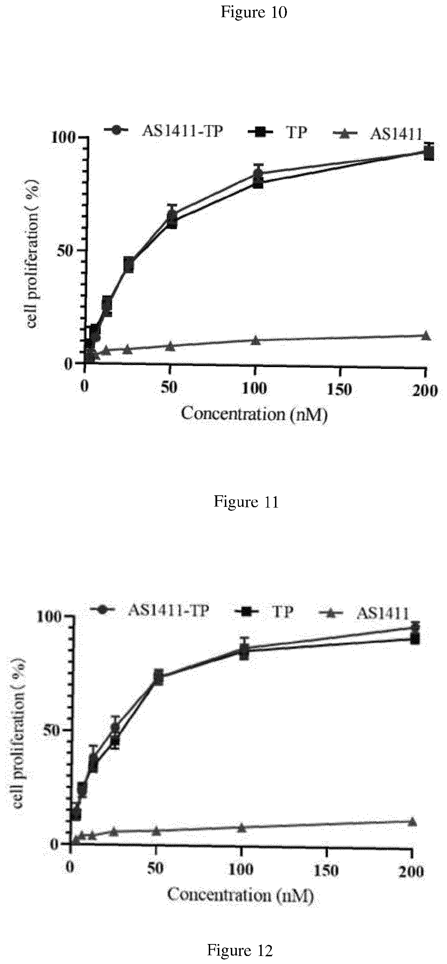

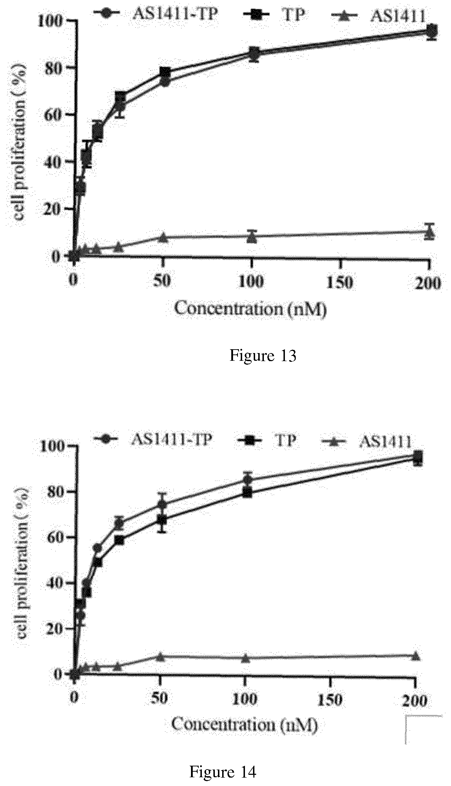

HCT116, MDA-MB-231, A549, PANC-1, and HepG2 cells with good growth conditions were cultured for serial passage, respectively. After resuspension, the cells were counted using a hemocytometer at a rate of 2500 cells/well, and then planted in a 96-well plate. After 24 hours of cell culture, the original medium was removed, while the medium containing different concentrations of drugs was added and incubated for additional 48 hours. After the drug action disappeared, the medium was discarded, and 100 µl of fresh medium was added, followed by addition of MTT solution (10 µl). The cells were cultured in an incubator for 2 hours, to which was added 150 ml of DMSO, and then allowed to shake in a shaker for 15 minutes. The OD value was measured using a microplate reader at 490 nm, and thus the inhibition rate of cell proliferation was calculated. The above steps were repeated three times. The inhibitory curves of AS1411-TP against the above 5 cancer cell lines are shown in Figures 10 to 14, and the experimental data are shown in Tables 1-5.

**Table 1. Summary table of the inhibition rates on the proliferation of colon cancer HCT116 cell lines treated with AS 1411-TP, TP, and AS 1411 for 48 hours.**

| Concentation (nM) | AS1411-TP | | | TP | | | AS1411 | | |
|---|---|---|---|---|---|---|---|---|---|
| | Sample 1 | Sample 2 | Sample 3 | Sample 1 | Sample 2 | Sample 3 | Sample 1 | Sample 2 | Sample 3 |
| 200.0 | 98.0396 | 99.3943 | 97.6337 | 97.2050 | 98.8058 | 98.5677 | 16.9608 | 13.5881 | 14.2937 |
| 100.0 | 83.9445 | 83.8214 | 79.5710 | 82.9707 | 80.3075 | 88.8251 | 10.6504 | 12.1637 | 10.9076 |
| 50.0 | 78.0639 | 74.8384 | 78.9549 | 75.5572 | 79.2127 | 76.2156 | 6.5424 | 6.5952 | 5.0330 |
| 25.0 | 56.0137 | 50.5940 | 54.8405 | 54.9296 | 66.2973 | 50.7426 | 5.4987 | 6.2971 | 5.6986 |
| 12.5 | 35.5002 | 38.0847 | 42.1452 | 42.1339 | 43.4099 | 47.0957 | 4.1987 | 3.0229 | 2.5102 |
| 6.3 | 22.9707 | 16.8472 | 30.6601 | 24.6824 | 20.0440 | 13.1188 | 2.9669 | 1.6683 | 3.1584 |
| 3.1 | 4.2221 | 5.5731 | 7.0957 | 9.8517 | 6.7785 | 3.8779 | 0.5424 | 1.4343 | 3.3666 |

**Table 2. Summary table of the inhibition rates on the proliferation of lung cancer A549 cell lines treated with AS 1411-TP, TP, and AS 1411 for 48 hours.**

| Concentratio n (nM) | AS1411-TP | | | TP | | | AS1411 | | |
|---|---|---|---|---|---|---|---|---|---|
| | Sample 1 | Sample 2 | Sample 3 | Sample 1 | Sample 2 | Sample 3 | Sample 1 | Sample 2 | Sample 3 |
| 200.0 | 93.5080 | 97.3771 | 96.2770 | 99.2234 | 97.0057 | 96.3263 | 14.5704 | 11.4171 | 14.3285 |
| 100.0 | 88.9080 | 81.4857 | 84.9945 | 80.0609 | 84.2971 | 83.2186 | 11.4727 | 11.8492 | 10.6369 |
| 50.0 | 69.8111 | 67.5314 | 62.1828 | 63.0205 | 66.0800 | 68.6718 | 9.0069 | 8.0722 | 7.6624 |
| 25.0 | 42.0455 | 42.0229 | 47.6989 | 41.4828 | 43.2000 | 46.9811 | 6.8129 | 6.6467 | 6.1306 |
| 12.5 | 20.6297 | 26.9486 | 27.6582 | 22.1328 | 29.4400 | 25.9563 | 5.5843 | 7.7330 | 4.4586 |
| 6.3 | 11.9297 | 10.7771 | 11.5908 | 13.6677 | 16.7429 | 15.2571 | 5.6975 | 3.3829 | 2.3885 |
| 3.1 | 4.1519 | 0.5029 | 4.9612 | 5.6673 | 3.5657 | 16.9811 | 3.2333 | 4.9611 | 2.2994 |

**Table 3. Summary table of the inhibition rates on the proliferation of breast cancer MDA-MB-231 cell lines treated with AS 1411-TP, TP, and AS 1411 for 48 hours.**

| Concentration (nM) | AS1411-TP | | | TP | | | AS1411 | | |
|---|---|---|---|---|---|---|---|---|---|
| | Sample 1 | Sample 2 | Sample 3 | Sample 1 | Sample 2 | Sample 3 | Sample 1 | Sample 2 | Sample 3 |
| 200.0 | 99.9080 | 97.4857 | 94.9945 | 98.0609 | 94.2971 | 93.2186 | 14.4727 | 11.8492 | 10.6369 |
| 100.0 | 89.8111 | 87.5314 | 82.1828 | 83.0205 | 86.0800 | 88.6718 | 9.0069 | 8.0722 | 8.6624 |
| 50.0 | 72.0455 | 72.0229 | 77.6989 | 71.4828 | 73.2000 | 76.9811 | 6.8129 | 6.6467 | 6.1306 |
| 25.0 | 50.6297 | 56.9486 | 47.6582 | 42.1328 | 49.4400 | 45.9563 | 5.5843 | 7.7330 | 4.4586 |
| 12.5 | 31.9297 | 40.7771 | 41.5908 | 31.6677 | 36.7429 | 35.2571 | 4.6975 | 5.3829 | 2.3885 |
| 6.3 | 24.1519 | 20.5029 | 24.9612 | 25.6673 | 23.5657 | 26.9811 | 3.2333 | 4.9611 | 4.2994 |
| 3.1 | 10.2234 | 17.0057 | 16.3263 | 14.5704 | 11.4171 | 14.3285 | 2.2910 | 2.1209 | 2.5159 |

**Table 4. Summary table of the inhibition rates on the proliferation of pancreatic cancer PANC-1 cell lines treated with AS 1411-TP, TP, and AS 1411 for 48 hours.**

| Concentration (nM) | AS1411-TP | | | TP | | | AS1411 | | |
|---|---|---|---|---|---|---|---|---|---|
| | Sample 1 | Sample 2 | Sample 3 | Sample 1 | Sample 2 | Sample 3 | Sample 1 | Sample 2 | Sample 3 |
| 200.0 | 98.1506 | 99.4049 | 96.2777 | 93.0451 | 98.8589 | 97.2313 | 9.6236 | 10.2679 | 9.9457 |
| 100.0 | 86.2970 | 89.0743 | 82.6857 | 80.3154 | 81.0105 | 79.6202 | 8.3147 | 7.1285 | 8.7216 |
| 50.0 | 72.5975 | 80.4612 | 71.5293 | 64.8389 | 65.1441 | 74.5337 | 7.2954 | 7.9552 | 9.6253 |
| 25.0 | 63.6216 | 69.1150 | 66.3683 | 58.9929 | 58.7555 | 59.2302 | 3.0417 | 4.2455 | 4.1946 |
| 12.5 | 56.5717 | 54.4015 | 55.4866 | 49.1929 | 47.8196 | 50.5663 | 3.6656 | 3.5639 | 3.6148 |
| 6.3 | 40.8545 | 39.1590 | 40.0068 | 35.9546 | 37.1753 | 34.7338 | 4.7169 | 1.9871 | 3.3520 |
| 3.1 | 30.7291 | 24.3744 | 22.5517 | 31.1631 | 30.2815 | 32.0448 | 0.3764 | 2.8325 | 3.9457 |

**Table 5. Summary table of the inhibition rates on the proliferation of liver cancer HepG2 cell lines treated with AS 1411-TP, TP, and AS 1411 for 48 hours.**

| Concentration (nM) | AS1411-TP | | | TP | | | AS1411 | | |
|---|---|---|---|---|---|---|---|---|---|
| | Sample 1 | Sample 2 | Sample 3 | Sample 1 | Sample 2 | Sample 3 | Sample 1 | Sample 2 | Sample 3 |
| 200.0 | 99.7291 | 94.3744 | 95.5517 | 95.1631 | 99.2815 | 99.0448 | 10.3764 | 15.8325 | 9.9457 |
| 100.0 | 87.8942 | 87.8467 | 82.8705 | 88.1738 | 88.3181 | 85.8901 | 7.2906 | 11.7328 | 8.7216 |
| 50.0 | 73.6283 | 75.2730 | 74.4507 | 78.5402 | 79.9729 | 77.1075 | 8.4300 | 6.9712 | 9.6253 |
| 25.0 | 61.8786 | 68.7080 | 60.2933 | 67.9620 | 66.7752 | 69.1489 | 4.1258 | 3.9268 | 4.1946 |
| 12.5 | 51.5632 | 57.2601 | 54.4117 | 48.6758 | 51.8210 | 55.5307 | 3.5280 | 2.4483 | 3.6148 |
| 6.3 | 39.1251 | 43.2621 | 41.1936 | 39.6846 | 39.8542 | 49.5151 | 1.8820 | 3.7911 | 3.3520 |
| 3.1 | 33.6216 | 29.1150 | 26.3683 | 28.9929 | 28.7555 | 29.2302 | 2.0417 | 2.2455 | 2.1946 |

Based on the data in Tables 1-5 above and the statistical graph of the inhibitory effects of the drugs on different cancer cells in Figures 3-7, the IC₅₀ values of triptolide against colon cancer HCT116 cells, breast cancer MDA-MB-231 cells, lung adenocarcinoma A549 cells, pancreatic cancer PANC-1 cells, and liver cancer HepG2 cells were 18.88 ± 0.13 nM, 22.38 ± 0.24 nM, 30.23 ± 0.17 nM, 14.4 ± 0.09 nM, and 10.46 ± 0.33 nM, respectively; the IC₅₀ values of AS1411-TP against colon cancer HCT116 cells, breast cancer MDA-MB-231 cells, lung adenocarcinoma A549 cells, pancreatic cancer PANC-1 cells, and liver cancer HepG2 cells were 20.01 ± 0.12 nM, 20.03 ± 0.27 nM, 29.87 ± 0.16 nM, 11.18 ± 0.03 nM, and 11.21 ± 0.26 nM, respectively. At the experimental concentration, AS 1411 showed no significant inhibitory effect on all tumor cells. The above data proved that AS1411-TP had anti-tumor effects on the above tumor cells, which were equivalent to that of triptolide, and was not caused by AS 1411. This indicated that the linkage to an aptamer did not affect the anti-tumor effect of triptolide, and at the same time, it was proved that the acid-sensitive linker responsive to the tumor microenvironment could cleave within tumor cells, releasing triptolide.

### III. In vitro toxicity study of AS1411-TP

The same materials as those in Part II above would not be repeated here. In addition, human normal liver LO2 cells and human embryonic kidney HEK293 cells were purchased from the cell bank of the Chinese Academy of Sciences (Shanghai) and stored in our laboratory.

### Experimental procedures

### 3.1 Cell recovery

Tumor cell lines LO2 and HEK293 were taken out from a liquid nitrogen tank, and placed in a 37 °C water bath. After the frozen cells melted into cell suspension, the suspension was centrifuged at a speed of 1000 r/min for 3 min, and then the supernatant was discarded, followed by adding 1 ml of fresh complete medium containing 10% fetal bovine serum and 1% double antibody. The cells were mixed well, and then 5 ml of medium was added. The cells were gently and repeatedly blown with a pipette to mix well. Finally, the cell suspension was transferred to a 10 mm culture dish, and after marking the cell name, cell passages, recovery date, and operator's name, the cells were placed in an incubator for cultivation (HEK293 cell lines were cultured in DMEM medium, while LO2 cell lines were cultured in RPMI1640 medium).

### 3.2 Cell passage

Well growing LO2 and HEK293 cells were selected, and then the medium was removed. The cells were washed with 1 ml of sterile PBS, to which was added 1.5 ml of trypsin to digest the cells, and after about 2 minutes, the cells become round and detached. 1.5 ml of medium was added to terminate the digestion, and then the cells were transferred to a centrifuge tube, and centrifuged at 1000 r/min for 3 min. The supernatant was discarded, 1 ml of fresh medium was added for resuspension, and then additional fresh medium was added in a ratio of 1:3 (v/v), followed by dividing into three culture dishes. The passage date and number were marked, and then the cells were placed in an incubator for cultivation.

### 3.3 Determining the cytotoxic activities of an aptamer-triptolide conjugate by MTT experiment

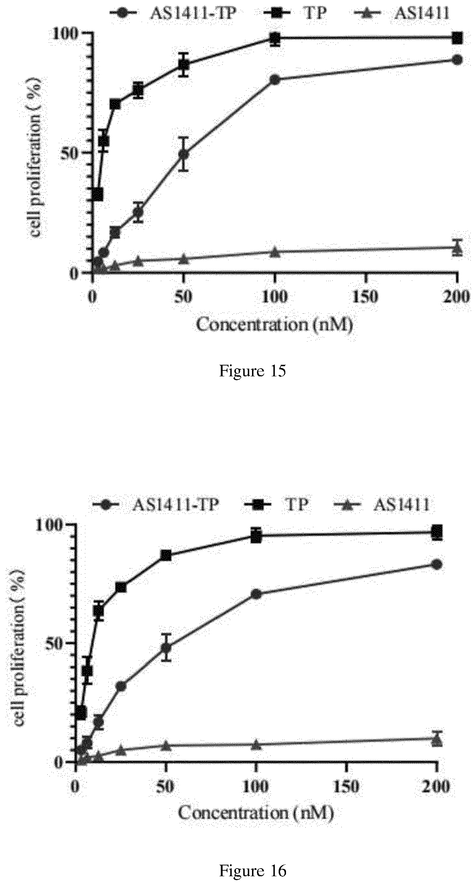

LO2 and HEK293 cells growing well were cultured for serial passage, respectively. After cell resuspension, the cells were counted using a hemocytometer at a rate of 3000 cells/well, and then seeded in a 96-well plate. After 24 hours of cell culture, the original medium was discarded, while the medium containing different concentrations of drugs was added for further culturing 48 hours. After the effects of drug disappeared, the medium was discarded, and 100 µl of fresh medium was added, followed by addition of MTT solution (10 µl). The cells were cultured in an incubator for 2 h, to which was added 150 ml of DMSO, and then allowed to shake in a shaker for 15 minutes. The OD value was measured using a microplate reader at 490 nm, and thus the inhibition rate of cell proliferation was calculated. The above steps were repeated three times. The inhibitory curves of AS1411-TP against normal liver LO2 cells and human embryonic kidney HEK293 cells are shown in Figures 15 to 16, and the experimental data are shown in Tables 6-7.

**Table 6. The inhibition rates on the proliferation of human embryonic kidney HEK293 cells treated with AS 1411-TP, TP, and AS 1411 for 48 hours.**

| Concentration (nM) | AS1411-TP | | | TP | | | AS1411 | | |
|---|---|---|---|---|---|---|---|---|---|
| | Sample 1 | Sample 2 | Sample 3 | Sample 1 | Sample 2 | Sample 3 | Sample 1 | Sample 2 | Sample 3 |
| 200.0 | 87.6596 | 89.6170 | 89.1489 | 98.2979 | 95.3901 | 100.6383 | 13.0496 | 11.5603 | 7.0200 |
| 100.0 | 79.2199 | 80.7801 | 81.4894 | 99.9291 | 94.3972 | 99.2908 | 9.5603 | 9.9858 | 6.6667 |
| 50.0 | 56.5937 | 43.2872 | 47.9317 | 89.5322 | 81.0372 | 89.5322 | 5.9341 | 4.5183 | 7.0200 |
| 25.0 | 29.6553 | 22.7331 | 23.1025 | 79.5322 | 73.0686 | 75.7495 | 4.6088 | 4.6251 | 5.5956 |
| 12.5 | 15.7481 | 15.4323 | 19.3525 | 69.2736 | 70.4830 | 71.1686 | 2.3262 | 2.9846 | 3.9285 |
| 6.3 | 8.5231 | 7.4099 | 9.3525 | 59.9053 | 53.1149 | 51.3265 | 1.4844 | 1.0633 | 2.0955 |
| 3.1 | 5.2744 | 4.1690 | 4.4492 | 35.0792 | 30.4489 | 32.7054 | 2.8090 | 1.3266 | 1.6489 |

**Table 7. The inhibition rates on the proliferation of human normal liver LO2 cells treated with AS 1411-TP, TP, and AS 1411 for 48 hours.**

| Concentration (nM) | AS1411-TP | | | TP | | | AS1411 | | |
|---|---|---|---|---|---|---|---|---|---|
| | Sample 1 | Sample 2 | Sample 3 | Sample 1 | Sample 2 | Sample 3 | Sample 1 | Sample 2 | Sample 3 |
| 200.0 | 81.6194 | 83.0397 | 85.1327 | 93.9952 | 99.8060 | 96.6312 | 8.3084 | 8.0987 | 13.5426 |
| 100.0 | 70.1522 | 69.0930 | 72.8414 | 96.2778 | 97.7552 | 82.0308 | 7.6842 | 6.8369 | 7.5344 |
| 50.0 | 54.6914 | 44.4723 | 45.0792 | 88.6995 | 87.4474 | 84.6990 | 7.3070 | 6.4174 | 7.0396 |
| 25.0 | 32.9996 | 30.9061 | 32.0759 | 74.1333 | 73.3084 | 73.3954 | 4.5962 | 5.2695 | 5.1506 |
| 12.5 | 20.2720 | 15.9757 | 14.3037 | 66.3653 | 59.2076 | 65.5783 | 3.1924 | 2.7446 | 2.0042 |
| 6.3 | 7.0403 | 6.2799 | 10.7730 | 31.9827 | 40.8401 | 42.6158 | 2.1198 | 1.5844 | 2.2503 |
| 3.1 | 5.5086 | 6.0304 | 3.7088 | 21.4060 | 23.3551 | 17.9111 | 0.4510 | 1.1866 | 0.8691 |

From the experimental data in Tables 6-7 above as well as the statistical graph of the inhibitory effects of the drugs on the proliferation of normal liver LO2 cells and normal human embryonic kidney HEK293 cells in Figures 8-9, it was shown that:
Triptolide had significant cytotoxicity on normal liver LO2 cells and normal human embryonic kidney HEK293 cells, with IC₅₀ values of 9.01 ± 0.25 nM and 5.98 ± 0.21 nM, respectively. When triptolide was conjugated with aptamer AS 1411, its cytotoxicity decreased, and the IC₅₀ values of the conjugate on LO2 and HEK293 cells were 51.86 ± 0.13 nM and 48.10 ± 0.13 nM, respectively. AS1411 had no significant inhibitory effect on both normal cells. This fully demonstrated that the linkage of the aptamer reduced the cytotoxicity of triptolide to normal cells.

There might be two reasons: 1) The aptamer blocked the active group of triptolide, and normal cells are in a non-weak-acid environment, making it impossible for triptolide to be fully released; 2) the expression level of nucleolin on the membrane of normal liver and kidney cells was relatively low, and aptamer AS 1411 could not target normal cells. Thereby, the intake of conjugates by normal cells was low, and thus the toxic effect was weak.

### IV. Investigating in vivo distribution of AS1411-TP

In order to investigate the *in vivo* distribution of AS 1411-TP conjugates, the mice models of colon cancer, breast cancer and lung cancer were used to carry out the distribution experiment *in vivo,* and the content of the conjugates in each tissue was measured by HPLC to study the distribution rule *in vivo.* The following was the *in vivo* distribution study performed in different tumor-bearing mice.

### 4.1 Colon cancer HCT116 cell lines

Material: The dosage of AS1411-TP group was set to be 50 µM, with a volume of 100 µL. The dosage of triptolide (TP) group was set to be 50 µM, with a volume of 100 µL. The same materials as in the second part above would not be repeated here, and in addition, the experimental animals also include BALB/cnu mice, if-, 6-7 weeks old, which is provided by SiPeiFu (Beijing) Biotechnology Co., Ltd., and raised in the experimental animal room of Chengdu University of Traditional Chinese Medicine.

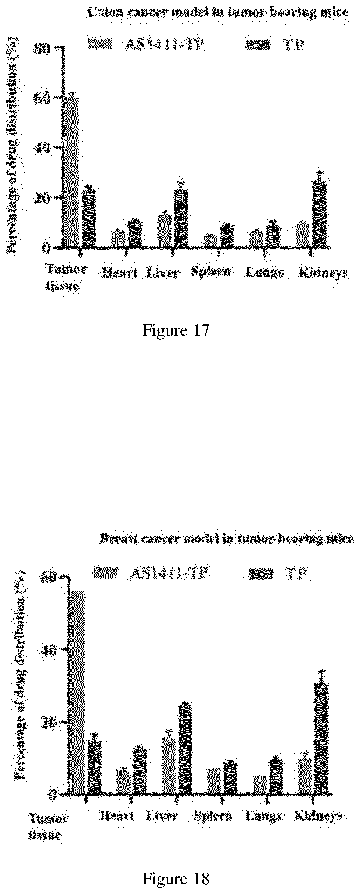

Experimental procedures: Female nude mice were raised in standard environment for 6-7 weeks. Colon cancer HCT116 cells were suspended in serum-free medium, and 3 * 10⁶ tumor cells were inoculated into each mouse. When the tumor volume reached 50-100 mm³, mice were randomly divided into two groups, with 12 mice in each group, and then AS1411-TP and TP were respectively injected into the tail vein of each mouse in corresponding group. 8 hours after administration, the animals were euthanized, and the heart, liver, spleen, lungs, kidneys, and tumor tissues were taken out. They were washed clean with physiological saline, weighed, and stored at -80 °C for short time. Each organ tissue was taken out, and then twice the amount of physiological saline was added to the tissues, followed by homogenizing, centrifuging and filtering. To 90 µL of filtrate, was added 10 µL of standard solution. After filtration, HPLC was used to determine the drug content in various tissues after treatment for different times. The distribution of the above drugs in different tissues of colon cancer models in tumor-bearing mice is shown in Figure 17.

According to the distribution results in Figure 17, AS1411-TP was more distributed in colon cancer tumor tissues, but less distributed in normal tissues; TP was more distributed in normal liver and kidney tissues. The content of AS1411-TP in normal mouse tissues was much lower than TP, while in tumor tissues, the AS1411-TP group had been found that a higher percentage of the total injection amount located in tumor tissues compared to triptolide group, indicating that AS1411-TP had good targeting ability for colon cancer tumor tissues *in vivo.*

### 4.2 Breast cancer MDA-MB-231 cell lines

The same materials as in part **4.1** would not be repeated in this experiment, and only the cancer cell lines had been changed.

Experimental procedures: Female nude mice were raised in standard environment for 6-7 weeks. Breast cancer MDA-MB-231 cells were suspended in serum-free medium, and 7 * 10⁶ tumor cells were inoculated into each mouse. When the tumor volume reached 50-100 mm³, mice were randomly divided into two groups, with 12 mice in each group, and then the aptamer-TP conjugate and TP were respectively injected into the tail vein of each mouse in corresponding group. 4 hours after administration, the animals were euthanized, and the heart, liver, spleen, lungs, kidneys, and tumor tissues were taken out. They were washed clean with physiological saline, weighed, and stored at -80 °C for short time. The contents of the aptamer-TP conjugate and TP in tissues were detected with HPLC. Each organ tissue was taken out, and then twice the amount of physiological saline was added to the tissues, followed by homogenizing, centrifuging and filtering. To 90 µL of filtrate, was added 10 µL of standard solution. After filtration, HPLC was used to determine the drug content in various tissues after treatment for different times. The distribution of the above drugs in different tissues of breast cancer models in tumor-bearing mice is shown in Figure 18.

According to the distribution in Figure 18, the AS 1411-TP conjugate was more distributed in breast cancer tumor tissues, but less distributed in normal tissues; TP was more distributed in normal liver and kidney tissues. The content of AS 1411-TP conjugate in normal mouse tissues was much lower than TP; while in tumor tissues, the AS1411-TP conjugate group had been found that a higher percentage of the total injection amount located in tumor tissues compared to triptolide group, indicating that the AS1411-TP conjugate had good targeting ability for breast cancer tumor tissues *in vivo.*

### 4.3 Lung cancer A549 cell lines

The same materials as in part **4.2** would not be repeated in this experiment, and only the cancer cell lines had been changed.

Experimental procedures: Female nude mice were raised in standard environment for 6-7 weeks. Lung cancer A549 cells were suspended in serum-free medium, and 6 * 10⁶ tumor cells were inoculated into each mouse. When the tumor volume reached 50-100 mm³, mice were randomly divided into two groups, with 12 mice in each group, and then the aptamer-TP conjugate and TP were respectively injected into the tail vein of each mouse in corresponding group. 4 hours after administration, the animals were euthanized, and the heart, liver, spleen, lungs, kidneys, and tumor tissues were taken out, washed clean with physiological saline, weighed, and stored at -80 °C for short time. The contents of the AS1411-TP conjugate and TP in tissues were detected with HPLC. The contents of the aptamer-TP conjugate and TP in tissues were detected with HPLC. Each organ tissue was taken out, and then twice the amount of physiological saline was added to the tissues, followed by homogenizing, centrifuging and filtering. To 90 µL of filtrate, was added 10 µL of standard solution. After filtration, HPLC was used to determine the drug content in various tissues after treatment for different times. The distribution of the above drugs in different tissues of lung cancer models in tumor-bearing mice is shown in Figure 19.

According to the distribution in Figure 19, the AS 1411-TP conjugate was more distributed in tumor tissues, but less distributed in normal tissues; TP was more distributed in normal liver and kidney tissues. The content of AS1411-TP conjugate in normal mouse tissues was much lower than TP; while in tumor tissues, the AS 1411-TP conjugate group had been found that a higher percentage of the total injection amount located in tumor tissues compared to triptolide group, indicating that the AS1411-TP conjugate had good targeting ability for tumors of lung cancer A549 cells *in vivo,* and simultaneously avoided other tissues, so as to have tumor-targeting effects.

### V. Investigation on the in vivo activity of AS1411-TP

In order to investigate the anti-tumor effect of the AS1411-TP conjugate *in vivo,* anti-tumor experiments were carried out in mice with colon cancer, breast cancer and lung cancer, respectively. The tumor, RTV value, tumor inhibition rate and T/C value were measured to prove its anti-tumor effect. The followings were *in vivo* activity studies performed using different tumor-bearing mice:

### 5.1 Colon cancer HCT116 cell lines

Female nude mice were raised in standard environment for 6-7 weeks. Colon cancer HCT116 cells were suspended in serum-free medium, and then 5 * 10⁶ tumor cells were inoculated into each mouse. When the tumor volume reached 50-100 mm³, mice were randomly divided into four groups, with 12 mice in each group, and then an aptamer-TP conjugate, TP, and the aptamer were respectively injected into the tail vein of each mouse in corresponding group. A PBS negative control group was included. TP (10 µM, 30 µM and 50 µM, with a volume of 100 µL), AS1411 (10 µM, 30 µM and 50 µM, with a volume of 100 µL), and AS1411-TP conjugate (10 µM, 30 µM and 50 µM, with a volume of 100 µL) were administered to respective test groups. The drug was given once every 2 days, and continuously administered for 8 times. The experiment was completed 24 hours after the last dose. After completion of the experiment, the animals were executed by breaking their cervical vertebra, and then the tumors were removed and weighed. The inhibition rate of drugs on tumor growth was calculated. The tumor weight, tumor volume, RTV and other indicators for each group of animals were compared using t-test method. The experimental data are summarized in Table 8, and Figure 20 shows the activities against colon cancer xenograft tumors in mice treated with different drugs.

**Table 8. Summary of experimental data for the inhibitory effect of AS 1411-TP, TP and AS 1411 on the growth of colon cancer models in tumor-bearing mice.**

| Groups | Tumor weight | Tumor inhibition rate (%) | RTV | T/C (%) |
|---|---|---|---|---|
| PBS | 3.11±0.21 | | 35.55±0.13 | |
| AS1411(10µM) | 2.92±0.33 | 6.45 | 33.13±0.51 | 93.19 |
| AS1411(30µM) | 2.74±0.15 | 12.91 | 28.56±0.23 | 80.34 |
| AS1411(50µM) | 2.66±0.14 | 16.12 | 26.32±0.15 | 74.04 |
| AS1411-TP(10µM) | 2.21±0.35 | 29.03 | 21.48±0.45 | 60.42 |
| AS1411-TP(30µM) | 1.54±0.37 | 51.61 | 15.76±0.15 | 44.33 |
| AS1411-TP(50µM) | 0.97±0.11 | 70.96 | 8.34±0.22 | 23.46 |
| TP(10µM) | 2.31±0.24 | 25.80 | 23.31±0.31 | 65.57 |
| TP(30µM) | 1.76±0.13 | 45.16 | 16.68±0.19 | 46.92 |
| TP(50µM) | 1.11±0.25 | 64.51 | 10.09±0.28 | 28.38 |

During the experimental process, the weight of the mice in the TP, AS 1411-TP, and AS 1411 groups remained in a tolerance range for toxicity and side effects during 8 doses. However, the weight of mice in PBS negative control group showed a slow and gradual decline trend, with an average weight reduction of 1.7 g compared to that when grouping. According to the tumor weight, inhibition rate, and RTV value of animals, the tumor growth rate and the tumor inhibition rate of mice in the TP, AS1411-TP, and AS1411 treatment groups were slowed down and showed a dose-dependent relationship compared to the PBS group. Among them, the AS1411-TP and TP groups showed a significant dose-response relationship for the tumor growth rate and the tumor inhibition rate of animals. But all indicators showed that the tumor inhibitory effect of TP group was lower than that of AS 1411-TP group at the corresponding concentration.

Experimental conclusion: when AS 1411-TP was injected into the tail vein of the colon cancer model in nude mice at a concentration of 10 µM, 30 µM and 50 µM for 8 times, respectively, the tumor growth was significantly inhibited, and the inhibitory activity was better than that of TP at the same concentration, indicating that AS 1411-TP had a good anti-tumor effect *in vivo.*

### 5.2 Breast cancer MDA-MB-231 cell lines

The materials used in the experiment were consistent with those in section **5.1** for test of colon cancer cell lines, with the only difference being that the tumor strains were different.

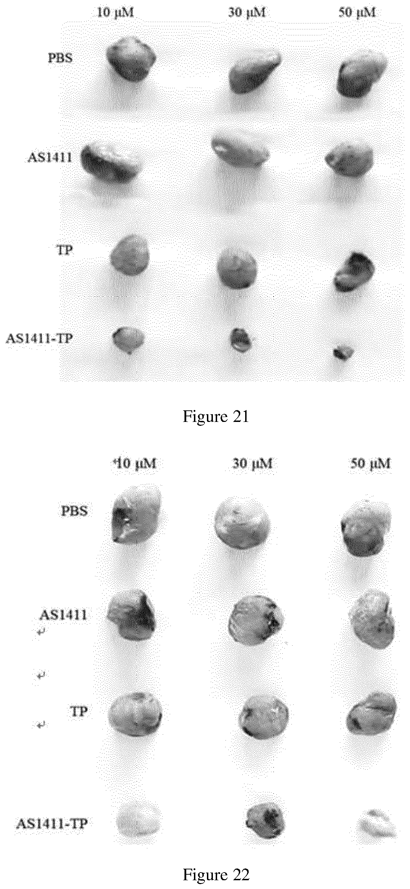

Specific experimental methods: Female nude mice were raised in standard environment for 6-7 weeks. Breast cancer MDA-MB-231 cell lines were suspended in serum-free medium, and then 6 * 10⁶ tumor cells were inoculated into each mouse. When the tumor volume reached 50-100 mm³, mice were randomly divided into four groups, with 12 mice in each group, and then an aptamer-TP conjugate, TP, and the aptamer were respectively injected into the tail vein of each mouse in corresponding group. A PBS negative control group was set up. TP (10 µM, 30 µM and 50 µM), AS1411 (10 µM, 30 µM and 50 µM),and AS1411-TP conjugate (10 µM, 30 µM and 50 µM) were administered to respective test groups. The drug was administrated once every 2 days, and continuously given for 8 times. The experiment was completed 24 hours after the last dose. After completion of the experiment, the animals were executed by breaking their cervical vertebra, and then the tumors were separated and weighed. The inhibition rate of drugs on tumor growth was calculated. The tumor weight, tumor volume, RTV and other indicators for each group of animals were compared using t-test method. The experimental data are summarized in Table 9, and Figure 21 shows the activities against breast cancer xenograft tumors in mice treated with different drugs.

**Table 9. Summary of experimental data for the inhibitory activities of AS 1411-TP, TP and AS 1411 on the growth of breast cancer models in tumor-bearing mice.**

| Groups | Tumor weight | Tumor inhibition rate (%) | RTV | T/C (%) |
|---|---|---|---|---|
| PBS | 3.43±0.27 | | 41.45±0.12 | |
| AS1411 (10 µM) | 3.19±0.27 | 7.00 | 39.29±16 | 94.79 |
| AS1411 (30 µM) | 2.92±0.39 | 14.87 | 37.55±34 | 90.59 |
| AS1411 (50 µM) | 2.78±0.41 | 18.95 | 35.19±38 | 84.90 |
| AS1411-TP (10 µM) | 2.13±0.17 | 37.90 | 24.53±45 | 59.18 |
| AS1411-TP (30 µM) | 1.41±0.11 | 58.89 | 13.56±14 | 32.71 |
| AS1411-TP (50 µM) | 0.88±0.26 | 74.34 | 9.77±22 | 23.57 |
| TP (10 µM) | 2.34±0.39 | 31.78 | 27.18±47 | 65.57 |
| TP (30 µM) | 1.77±0.51 | 48.40 | 16.44±31 | 39.66 |
| TP (50 µM) | 0.89±0.08 | 74.05 | 12.89±55 | 31.10 |

During the experimental process, the weight of the mice in the TP, AS1411-TP, and AS1411 groups remained in a tolerance range for animal toxicity and side effects during 8 doses. However, the weight of mice in PBS negative control group showed a slow and gradual decline trend, with an average weight reduction of 2.1 g compared to that when grouping. According to the tumor weight, inhibition rate, and RTV value of animals, the tumor growth rate and the tumor inhibition rate of mice in the TP, AS1411-TP, and AS1411 treatment groups were slowed down and showed a dose-dependent relationship compared to the PBS group. Among them, the AS1411-TP and TP groups showed a significant dose-response relationship for the tumor growth rate and the tumor inhibition rate of animals. But all indicators showed that the tumor inhibitory effect of TP group was lower than that of AS 1411-TP group at the corresponding concentration.

Based on the results in Table 9 and Figure 21, it was indicated that when AS1411-TP was injected into the tail vein of the breast cancer model in nude mice at a concentration of 10 µM, 30 µM and 50 µM for 8 times, respectively, the growth of breast cancer cells was significantly inhibited. The inhibition rate was obviously related to the dosage, and the inhibitory activity was better than that of TP at the same concentration, indicating that AS 1411-TP had a good *in vivo* effect on breast cancer.

### 5.3 Lung cancer A549 cell lines

Experimental methods: Female nude mice were raised in standard environment for 6-7 weeks. Lung cancer A549 cell lines were suspended in serum-free medium, and then 6 * 10⁶ tumor cells were inoculated into each mouse. When the tumor volume reached 50-100 mm³, mice were randomly divided into four groups, with 12 mice in each group, and then an AS1411-TP conjugate, TP, and the aptamer were respectively injected into the tail vein of each mouse in corresponding group. APBS negative control group was set up. TP (10 µM, 30 µM and 50 µM),AS1411 (10 µM, 30 µM and 50 µM),and AS1411-TP conjugate (10 µM, 30 µM and 50 µM) were administered to respective test groups. The drug was administrated once every 2 days, and continuously dosed for 8 times. The experiment was completed 24 hours after the last dose. After completion of the experiment, the animals were executed by breaking their cervical vertebra, and then the tumors were separated and weighed. The inhibition rate of drugs on tumor growth was calculated. The tumor weight, tumor volume, RTV and other indicators for each group of animals were compared using t-test method. The experimental data are summarized in Table 10, and Figure 22 shows the activities against lung cancer xenograft tumors in mice treated with different drugs.

**Table 10. Summary of experimental data for the inhibitory activities of AS 1411-TP, TP and AS1411 on the growth of lung cancer models in tumor-bearing mice.**

| Groups | Tumor weight | Tumor inhibition rate (%) | RTV | T/C (%) |
|---|---|---|---|---|
| PBS | 3.33±0.34 | | 37.13±0.11 | |
| AS1411 ( 10 µM ) | 3.16±0.13 | 6.06 | 34.57±0.18 | 89.93 |
| AS1411 (30µM) | 2.88±0.26 | 15.15 | 31.09±0.27 | 75.76 |
| AS1411 (50 µM) | 2.61±0.38 | 21.21 | 26.19±0.38 | 70.06 |
| AS1411-TP (10 µM) | 2.29±0.27 | 33.33 | 24.22±0.55 | 40.79 |
| AS1411-TP (30 µM) | 1.51±0.19 | 54.55 | 14.1±0.26 | 26.55 |
| AS1411-TP (50 µM) | 1.15±0.16 | 66.67 | 9.18±0.37 | 75.50 |
| TP (10 µM) | 2.47±0.08 | 27.27 | 26.1±0.09 | 51.81 |
| TP (30 µM) | 1.79±0.19 | 48.48 | 17.91±0.27 | 31.59 |
| TP (50 µM) | 1.29±0.27 | 63.64 | 10.92±0.15 | 89.93 |

During the experimental process, the weight of the mice in the TP, AS 1411-TP, and AS 1411 groups remained in a tolerance range for toxicity and side effects during 8 doses. However, the weight of mice in PBS negative control group showed a slow and gradual decline trend, with an average weight reduction of 2.0 g compared to that when grouping. According to the tumor weight, inhibition rate, and RTV value of animals, the tumor growth rate and the tumor inhibition rate of mice in the TP, AS1411-TP, and AS1411 treatment groups were slowed down and showed a dose-dependent relationship compared to the PBS group. Among them, the AS1411-TP and TP groups showed a significant dose-response relationship for the tumor growth rate and the tumor inhibition rate of animals. But all indicators showed that the tumor inhibitory effect of TP group was lower than that of AS 1411-TP group at the corresponding concentration.

Based on the results in Table 10 and Figure 22, it was shown that when AS1411-TP was injected into the tail vein of nude mice bearing lung adenocarcinoma A549 cell lines at a concentration of 10 µM, 30 µM and 50 µM for 8 times, respectively, the growth of lung cancer A549 cell lines was significantly inhibited. The inhibition rate was obviously related to the dosage, and the inhibitory activity was better than that of TP at the same concentration, indicating that AS 1411-TP had a good targeted inhibitory effect on lung adenocarcinoma.

### VI. Study on the in vivo stability of AS1411-TP

To simulate the stability of the drug in the human body and clarify its dissociation and stability in different tissues, stability experiments of an aptamer-triptolide conjugate were carried out in mice.

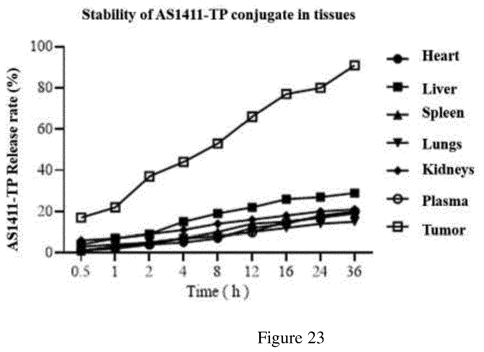

Experimental method: 27 tumor-bearing mice were randomly selected, and the solution of AS1411-TP conjugate in physiological saline (50 µM, 100 µL) was injected into the tail vein of each mouse. At 0.5 h, 1 h, 2 h, 4 h, 8 h, 12 h, 16 h, 24 h, and 32 h, three mice were anesthetized by intraperitoneal injection of pentobarbital sodium and euthanized, respectively. Blood was collected via cardiac puncture, and tissues such as the heart, liver, spleen, lungs, kidneys, and solid tumors were taken out, homogenized and extracted. After determining the retention times of TP, AS 1411, and AS1411-TP conjugates by sample processing and pre-experiments, the amounts of free TP and AS1411-TP in each tissue were detected with HPLC at each time point, and the release rate was calculated. The stability of the drug in the tissue was monitored and quantitatively evaluated. The experimental data are shown in Table 11, and the analysis results of *in vivo* stability experimental data are shown in Figure 23.

**Table 11. In vivo stability experimental data of an aptamer-triptolide conjugate (AS 1411-TP).**

| Drug release rates from different tissues at different times | 1/2 h | 1 h | 2 h | 4 h | 8 h | 12 h | 16 h | 24 h | 36 h |
|---|---|---|---|---|---|---|---|---|---|
| Heart | 1% | 2% | 4% | 5% | 7% | 12% | 14% | 18% | 20% |
| Liver | 4% | 7% | 9% | 15% | 19% | 22% | 26% | 27% | 29% |
| Spleen | 1% | 3% | 5% | 7% | 10% | 14% | 15% | 17% | 19% |
| Lungs | 3% | 4% | 5% | 7% | 8% | 10% | 12% | 14% | 15% |
| kidneys | 6% | 7% | 9% | 11% | 14% | 16% | 18% | 20% | 21% |
| Blood | 1% | 3% | 4% | 7% | 8% | 10% | 15% | 17% | 20% |
| Tumor | 17% | 22% | 37% | 44% | 53% | 66% | 77% | 80% | 91% |

Figure 23 shows the analysis graph of *in vivo* stability experimental data for an aptamer-triptolide conjugate (AS1411-TP). Based on the data in Table 11 and Figure 23, it could be shown that after 36 hours, the AS1411-TP conjugate was almost not dissociated or only slightly dissociated in normal tissues such as plasma, heart, spleen, and lungs, as well as slightly more dissociated in kidney and liver tissues; however, in tumor tissues, lots of AS1411-TP conjugates were found to cleave, and the dissociation degree increased over time. The above results demonstrated that the linker used to connect triptolide and an aptamer played a key role *in vivo.* The linker ensured that the conjugate did not break in normal tissues and had low toxicity; and when the conjugate cleaved in tumor tissues, it released triptolide, which played a therapeutic role.

Conclusion: In summary, according to the above experiments on the drugs, the aptamer-triptolide conjugate provided in the present invention was linked by a linker containing an acetal ester moiety. Compared with the linker containing a vinyl ether moiety in the prior art, the present linker had better weak-acid responsiveness, with a cleavage pH of 3.5-6.5, while the cleavage condition of the vinyl ether moiety was below pH=3.5. Therefore, for the tumor microenvironment, the aptamer-triptolide conjugate provided in the present invention was more responsive to the tumor microenvironment. At the same time, *in vitro* experiments had shown that the aptamer-triptolide conjugate provided in the present invention had better serum stability.

The aptamer-triptolide conjugate provided in the present invention had anti-tumor effects comparable to triptolide both *in vivo* and *in vitro,* which proved that the connection of the aptamer did not affect the anti-tumor effect of the conjugate. Meanwhile, both *in vitro* and *in vivo* experiments had shown that the toxicity of the conjugate was reduced compared to triptolide, indicating that the conjugate had better targeting anti-tumor effects and could avoid toxicity to normal tissues.

The distribution of the aptamer-triptolide conjugate provided in the present invention was much higher in tumor tissues than in other tissues, and the dissociation rate in tumor tissues was much higher than in normal tissues. This proved that the conjugate provided in the present invention had good tumor targeting *in vivo,* and the tumor microenvironment could meet the release conditions of the conjugate.

## Claims

1. A triptolide conjugate, **characterized in that** the general structural formula of the conjugate is as follows: 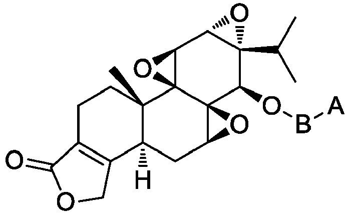 wherein, A is an aptamer;
B is a linker between triptolide and an aptamer, and the linker comprises an acetal ester moiety formed at the 14-OH position.

2. A triptolide analogue, **characterized in that** the general structural formula of the conjugate is as follows: 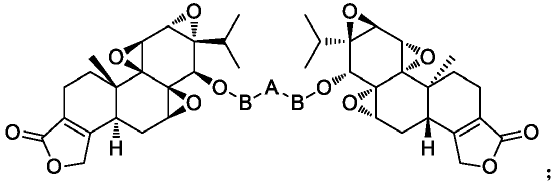 wherein, A is an aptamer;
B is a linker between triptolide and an aptamer, and the linker comprises an acetal ester moiety formed at the 14-OH position.

3. An acid-sensitive conjugate of an aptamer and triptolide according to claim 1 or 2, **characterized in that** the general structural formula of the linker, i.e. B, is as follows:

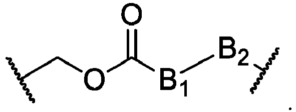

4. An acid-sensitive conjugate of an aptamer and triptolide according to claim 3, **characterized in that** the general structural formula of B₁ is: 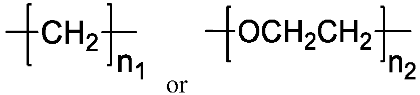 or a combination thereof;
wherein, 1≤n₁≤100; preferably, 1≤n₁≤50; more preferably, 1≤n₁≤20;
wherein, 1≤n₂≤100; preferably, 1≤n₂≤50; more preferably, 1≤n₂≤20.

5. An acid-sensitive conjugate of an aptamer and triptolide according to claim 3, **characterized in that** the general structural formula of B₂ is as follows:

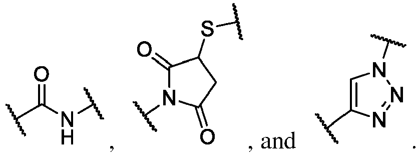

6. An acid-sensitive conjugate of an aptamer and triptolide according to any one of claim 1 or 2, **characterized in that** the aptamer includes an aptamer modifier, and the general structural formula of the aptamer modifier is as follows: A1-C1-; the structure of C1 includes: 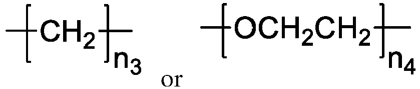 or a combination thereof;
wherein, 1≤n₃≤100; preferably, 1≤n₃≤50; more preferably, 1≤n₃≤20;
wherein, 1≤n₄≤100; preferably, 1≤n₄≤50; more preferably, 1≤n₄≤20.

7. An acid-sensitive conjugate of an aptamer and triptolide according to claim 6, **characterized in that** A1 is selected from the group consisting of AS 1411, Pegaptanib, Sgc8c, A10, DNAaptamer, RNAaptamer, CL4, Apt, and E07.

8. An acid-sensitive conjugate of an aptamer and triptolide according to claim 7, **characterized in that**:
the sequence of A1 is as follows:
AS 1411: GGTGGTGGTGGTTGTGGTGGTGGTGG;
Pegaptanib: GCGAACCGAUGGAAUUUUUGGACGCUCGC;
Sgc8c: ATCTAACTGCTGCGCCGCCGGGAAAATACTGTACGGTTAGA;
A10: 
GGGAGGACGAUGCGGAUCAGCCAUGUUUACGUCACUCCUUGUCAAUCCUCAUCGGC
AGACGACUCGCCCGA;
DNAaptamer: GGGAGACAAGAATAAACGCTCAA-(25N)-TTCGACAGGAGGCTCACAACAGGC;
RNAaptamer: GGGGGCAUACUUGUGAGACUUUUAUGUCACCCCC;
CL4: GCCUUAGUAACGUGCUUUGAUGUCGAUUCGACAGGAGGC;
Apt: GCAGTTGATCCTTTGGATACCCTGG;
E07: GGACGGAUUUAAUCGCCGUAGAAAAGCAUGUCAAAGCCGGAACCGUCC.

9. A method for preparing the acid-sensitive conjugate of an aptamer and triptolide according to any one of claims 1-8, **characterized in that** the method comprises the following steps:
Step 1: A triptolide analogue is prepared, wherein the 14-OH position of triptolide is linked to an ester group;
Step 2: The aptamer is modified to obtain the aptamer modifiers Ai-Ci;
Step 3: The conjugate is synthesized by linking a triptolide analogue and an aptamer modifier obtained in step 2 with the linker B₂.

10. The conjugate according to any one of claims 1 to 9 for use in the manufacturer of anti-tumor medicaments.
